(19) Europäisches Patentamt
European Patent Office
Office européen des brevets

(11) Publication number: 0 127 446
B1

(12) EUROPEAN PATENT SPECIFICATION

(45) Date of publication of patent specification: 05.08.87

(51) Int. Cl.⁴: C 07 D 233/90, A 01 N 43/50

(21) Application number: 84303520.5

(22) Date of filing: 24.05.84

# (54) Esters of 2-bromo-4-methylimidazole-5-carboxylic acid.

(30) Priority: 01.06.83 US 499955
14.03.84 US 589608

(43) Date of publication of application:
05.12.84 Bulletin 84/49

(45) Publication of the grant of the patent:
05.08.87 Bulletin 87/32

(84) Designated Contracting States:
AT BE CH DE FR GB IT LI NL

(58) References cited:
FR-A-2 336 398

CHEMICAL ABSTRACTS, volume 17, no. 11, 10th June 1923, page 1964, (COLUMBUS, OHIO, US) F.L. PYMAN et al. "Bromo derivatives of 4-methylglyoxaline"

(73) Proprietor: STAUFFER CHEMICAL COMPANY
Westport Connecticut 06881 (US)

(72) Inventor: Bayer, Arthur Craig
3 Somerston Road
Yorktown Heights New York 10598 (US)

(74) Representative: Smith, Sydney et al
Elkington and Fife High Holborn House
52/54 High Holborn
London WC1V 6SH (GB)

EP 0 127 446 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to imidazole esters of use as herbicides.

Compounds having the structural formula:

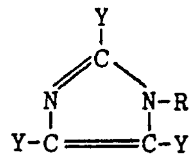

wherein R is hydrogen, alkyl or substituted alkyl and Y is hydrogen, alkyl, substituted alkyl, halogen, cyano or nitro are described in U.S. Patent No. 3,501,286 as being herbicides.

A compound of the formula:

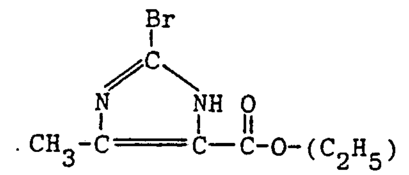

is taught by Pyman and Timmis, *J. Chem. Soc.*, pp. 494—498 (1923). However, no utility for this compound is taught other than its use as an intermediate in the preparation of pharmaceuticals.

DESCRIPTION OF THE INVENTION

The invention relates to esters of 2-bromo-4-methylimidazole-5-carboxylic acid as herbicides. The novel compounds of this invention have the following structural formula:

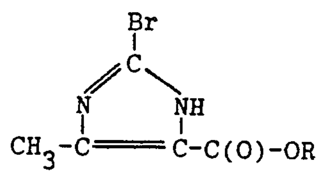

wherein R is methyl or $C_3$—$C_{10}$ alkyl, preferably $C_3$—$C_6$ alkyl, more preferably $C_3$—$C_5$ alkyl, most preferably isopropyl, isobutyl, isopentyl, or sec-pentyl; $C_3$—$C_8$ cycloalkyl, preferably $C_4$—$C_6$ cycloalkyl, more preferably cyclopentyl or cyclohexyl; ($C_3$—$C_8$ cycloalkyl)alkyl wherein the alkyl part has 1 to 8 carbon atoms, preferably cyclopropylmethyl or cyclopentylmethyl; $C_3$—$C_{10}$ alkenyl, preferably allyl or 2-methyl-3-butenyl-1; $C_5$—$C_8$ cycloalkenyl; benzyl or mono- or di-substituted benzyl wherein the substituents are $C_1$—$C_4$ alkyl, halogen, nitro or $C_1$—$C_4$ haloalkyl. More preferably R is $C_3$—$C_6$ alkyl. The compound where R is ethyl is known, however, its use as an herbicide is novel.

In the above description of the compounds of this invention alkyl includes both straight and branched configurations; for example, methyl, ethyl, n-propyl, isopropyl, n-butyl, sec-butyl, isobutyl, and tert-butyl, the amyls, the hexyls, the heptyls, the nonyls and the decyls.

The compounds of this invention are active herbicides of a general type. That is, they are herbicidally effective against a wide range of plant species. The method of controlling undesirable vegetation of the present invention comprises applying an herbicidally effective amount of the above-described compounds to the area where control is desired.

The compounds of the present invention can be prepared by the following general method.

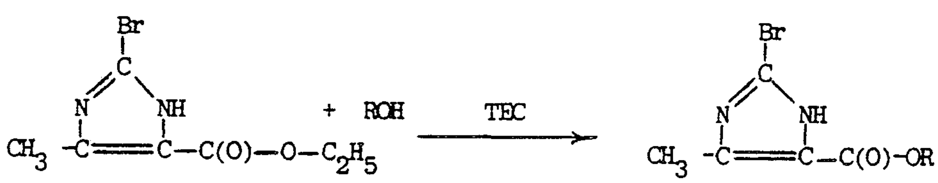


wherein R is as defined, and TEC is a transesterification catalyst such as Ti(O-alkyl)$_4$ preferably Ti (isobutoxy)$_4$.

The compounds of this invention can have the following two structural formulae because of tautomerism, that is, the proton on the imidazole nitrogen can reside on eigher nitrogen and is reasonably labile. Structural formula I is the tautomer which is believed to predominate in the solid state since the infrared spectra show longer-wave-length carbonyl bands compared to ordinary esters. This fact suggests hydrogen bonding is possible only in the tautomer I.

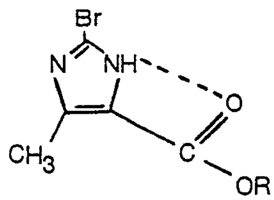

or

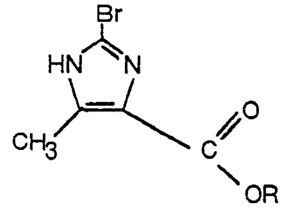

I II

Both tautomers I and II are believed to be in mobile equilibrium in solutions or in the liquid state.

The proton on tautomers I and II is acidic and can be removed by any base to give a salt having an anion of the following two resonance forms:

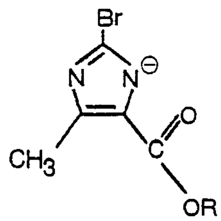

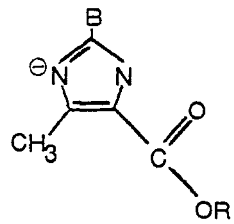

Ia IIa

Examples of cations of these bases can be an inorganic cation such as $Na^+$, $K^+$, $Ca^{++}$, $NH_4^+$ or it can be an organic cation such as hydrocarbyl-substituted ammonium, sulfonium, or phosphonium.

These salts are stable compounds and can be isolated. They are also herbicidally active.

The imidazoles of the invention are also basic. The unprotonated nitrogen atom of tautomer Ia or IIa can be protonated by an acid, preferably an inorganic acid. Illustrative inorganic acids are hydrochloric and hydrobromic, sulfuric, phosphoric and acetic. These salts are also herbicidal.

The salts can be conventionally prepared by reacting at least a mole amount of a Lewis acid or a Lewis base with the ester. Preferably, the reaction is run in a solvent for the ester with heating, if necessary. The prepared salt is recovered from the reaction mixture by conventional techniques.

In general, the salts are not as herbicidally active as the parent compound.

Generally, at least one mole of the alcohol is used for the reaction with the ethyl ester to prepare the imidazoles. Preferably, a slight mole excess of the alcohol is used. The reaction mixture is refluxed until completion of the reaction. The reaction product is recovered by removing the volatile materials. Atmospheric, subatmospheric or superatmospheric pressures can be used, depending on the boiling point of the solvent used. Ethanol is conveniently stripped at elevated temperatures and reduced pressure.

The reaction product is worked up by conventional techniques.

The following example teaches the synthesis of a representative compound of this invention.

Example I

Isopropyl ester of 2-Bromo-4-methyl-5-imidazolecarboxylic acid

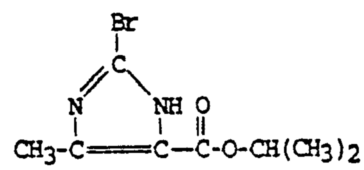

6.4 grams (g), 0.027 mole of the ethyl ester of 2-bromo-4-methyl-5-imidazole carboxylic acid, 70 cubic centimeters (cc) isopropanol, and 6 drops tetraisobutyl titanate were refluxed under a nitrogen atmosphere for ten days. The reaction mixture was stripped to 90°C and 1 mm kg causing crystallization. The crystals were filtered and recrystallized from isopropanol to yield 3.4 g of the desired product, a white crystalline solid, m.p. 193.5—194.5. The structure was confirmed by nuclear magnetic resonance and infrared spectroscopy.

Example II

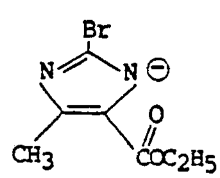

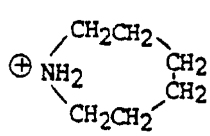

To 4.0 g (0.017 mole) of the ethyl ester of 2-bromo-4-methyl-5-imidazolecarboxylic acid is added dropwise 4.0 g (0.4 mole) of hexamethyleneimine. The mixture was stirred at 23°C for 2 hours, then stripped to 40°C and 2 mm Hg, causing crystallization of the desired product, a white crystalline solid, m.p. 94—94.5°C. The structure was confirmed by IR and $^1$HNMR analyses.

The following is a table of certain selected compounds that are preparable according to the procedure described herein. Compound numbers are assigned to each compound and are used throughout the remainder of the application.

TABLE I

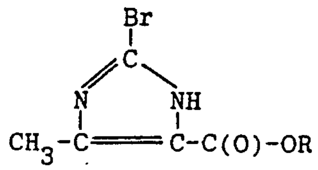

| Compound Number | R | $n_D^{25}$ or m.p. °C |
|---|---|---|
| 1 | ethyl | light yellow solid |
| 2 | i-propyl | 193.5—194.5 |
| 3 | n-butyl | glassy solid |
| 4 | sec-butyl | 140—141 |
| 5 | i-butyl | 111.5—112.5 |
| 6 | n-hexyl | semi-solid |
| 7 | n-octyl | 1.5136 |
| 8 | n-decyl | 1.4999 |
| 9 | 3-methylbutyl | glassy solid |
| 10 | cyclohexyl | 51—53.5 |
| 11 | cyclopentyl | 173—174.5 |
| 12 | n-propyl | 108.5—110.5 |
| 13 | 1-methylbutyl | 119—120 |
| 14 | 1-ethylpropyl | 178—180 |
| 15 | n-pentyl | 93—94.5 |
| 16 | 1,3-dimethylbutyl | 146—146.5 |
| 17 | methyl | 145.5—147 |
| 18 | t-butyl | golden oil |
| 19 | cyclopropylmethyl | yellow oil |
| 20 | 2,2-dimethylpropyl | dark oil |
| 21 | 1-methyl-3-propenyl | golden oil |
| 22 | cyclopentylmethyl | golden oil |
| 23 | sodium salt of Compound No. 19 | |

TABLE I (continued)

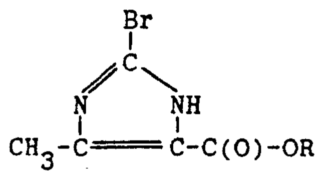

| Compound Number | R | $n_D^{25}$ or m.p. °C |
|---|---|---|
| 24 | hexamethylene imine salt of Compound No. 2 | 117—119 |
| 25 | tetra-n-butyl ammonium salt of Compound No. 2 | semi-solid |
| 26 | sodium salt of Compound No. 2 | dec. 255 |
| 27 | hexamethylene imine salt of Compound No. 1 | 94—94.5 |

Herbicidal Screening Tests

As previously mentioned, the herein described compounds produced in the above-described manner are phytotoxic compounds which are useful and valuable in controlling various plant species. Selected compounds of this invention were tested as herbicides in the following manner.

*Pre-emergence herbicide test.* On the day preceding treatment, seeds of eight different weed species are planted in loamy sand soil in individual rows using one species per row across the width of a flat. The seeds used are green foxtail (FT) (*Setaria viridis*), watergrass (WG) (*Echinochloa crusgalli*), wild oat (WO) (*Avena fatua*), annual morningglory (AMG) (*Ipomoea lacunosa*), velvetleaf (VL) (*Abutilon theophrasti*), Indian mustard (MD) (*Brassica juncea*), curly dock (CD); (*Rumex crispus*), and yellow nutsedge (YNG) (*Cyperus esculentus*). Ample seeds are planted to give about 20 to 40 seedlings per row, after emergence, depending upon the size of the plants.

Using an analytical balance, 600 milligrams (mg) of the compound to be tested are weighed out on a piece of glassine weighing paper. The paper and compound are placed in a 60 milliliter (ml) wide-mouth clear bottle and dissolved in 45 ml of acetone or substituted solvent. Eighteen ml of this solution are transferred to a 60 ml wide-mouth clear bottle and diluted with 22 ml of a water and acetone mixture (19:1) containing enough polyoxyethylene sorbitan monolaurate emulsifier to give a final solution of 0.5% (v/v). The solution is then sprayed on a seeded flat on a linear spray table calibrated to deliver 80 gallons per acre (748 L/ha). The application rate is 4 lb/acre (4.48 Kg/ha).

After treatment, the flats are placed in the greenhouse at a temperature of 70 to 80°F (21 to 27°C) and watered by sprinkling. Two weeks after treatment, the degree of injury or control is determined by comparison with untreated check plants of the same age. The injury rating from 0 to 100% is recorded for each species as percent control with 0% representing no injury and 100% representing complete control.

The results of the tests are shown in the following Table II.

TABLE II
Pre-Emergence Herbicidal Activity
Application Rate — 4.48 kg/ha

| Compound No. | FT | WG | WO | AMG | VL | MD | CD | YNG |
|---|---|---|---|---|---|---|---|---|
| 1 | 10 | 20 | 20 | 20 | 30 | 60 | 20 | 0 |
| 2 | 100 | 100 | 100 | 100 | 100 | 100 | 100 | 20 |
| 3 | 0 | 20 | 0 | 35 | 0 | 20 | 90 | 0 |
| 4 | 100 | 100 | 50 | 90 | 100 | 100 | 90 | 0 |
| 5 | 20 | 20 | 20 | 10 | 10 | 10 | 100 | 0 |
| 6 | 0 | 0 | 0 | 0 | 0 | 0 | 0 | 0 |
| 7 | 80 | 40 | 20 | 0 | 0 | 0 | 0 | 0 |

TABLE II (continued)

Pre-Emergence Herbicidal Activity
Application Rate — 4.48 kg/ha

| Compound No. | FT | WG | WO | AMG | VL | MD | CD | YNG |
|---|---|---|---|---|---|---|---|---|
| 8 | 0 | 0 | 0 | 0 | 0 | 0 | 0 | 0 |
| 9 | 0 | 0 | 0 | 0 | 0 | 0 | 0 | 0 |
| 10 | 0 | 0 | 0 | 0 | 0 | 0 | 0 | 0 |
| 11 | 0 | 20 | 0 | 40 | 70 | 60 | 60 | 0 |
| 12 | 10 | 30 | 10 | 20 | 10 | 30 | 60 | 0 |
| 13 | 100 | 80 | 80 | 80 | 100 | 100 | 100 | 0 |
| 14 | 100 | 100 | 90 | 90 | 100 | 100 | 100 | 0 |
| 15 | 0 | 0 | 0 | 0 | 0 | 0 | 0 | 0 |
| 16 | 0 | 0 | 0 | 0 | 0 | 0 | 0 | 0 |
| 17 | 0 | 0 | 0 | 0 | 0 | 0 | 0 | 0 |
| 18 | 100 | 90 | 45 | 100 | 100 | 100 | 80 | 0 |
| 19 | 20 | 20 | 0 | 60 | 60 | 60 | 80 | 0 |
| 20 | 0 | 0 | 0 | 30 | 100 | 100 | 0 | 0 |
| 21 | 80 | 80 | 40 | 60 | 90 | 90 | 60 | 0 |
| 22 | 0 | 0 | 0 | 0 | 0 | 0 | 0 | 0 |
| 23 | 0 | 0 | 0 | 80 | 80 | 80 | 0 | 0 |
| 24 | 90 | 70 | 20 | 60 | 85 | 60 | 80 | 0 |
| 25 | 40 | 60 | 10 | 20 | 30 | 40 | 60 | 0 |
| 26 | 80 | 65 | 10 | 40 | 80 | 85 | 85 | 0 |
| 27 | 20 | 40 | 20 | 50 | 10 | 90 | 90 | 0 |

*Post-Emergence Herbicide Test:* This test is conducted in an identical manner to the testing procedure for the pre-emergence herbicide test, except the seeds of the eight different weed species are planted 10—12 days before treatment. Also, watering of the treated flats is confined to the soil surface and not to the foliage of the sprouted plants.

The results of the post-emergence herbicide test are reported in Table III.

TABLE III
Post-Emergence Herbicidal Activity
Application Rate — 4.48 kg/ha

| Compound No. | FT | WG | WO | AMG | VL | MD | CD | YNG |
|---|---|---|---|---|---|---|---|---|
| 1 | 85 | 85 | 85 | 100 | 100 | 100 | 100 | 0 |
| 2 | 100 | 100 | 100 | 100 | 100 | 100 | 100 | 40 |
| 3 | 100 | 100 | 100 | 95 | 100 | 100 | 100 | 0 |
| 4 | 100 | 100 | 100 | 100 | 100 | 100 | 100 | 10 |
| 5 | 100 | 60 | 60 | 80 | 100 | 100 | 100 | 0 |
| 6 | 60 | 20 | 20 | 80 | 80 | 90 | 80 | 0 |
| 7 | 80 | 40 | 20 | 0 | 0 | 80 | 0 | 0 |
| 8 | 0 | 0 | 0 | 10 | 90 | 90 | 30 | 0 |
| 9 | 30 | 20 | 10 | 30 | 40 | 40 | 50 | 0 |
| 10 | 100 | 60 | 90 | 40 | 100 | 100 | 100 | 0 |
| 11 | 100 | 60 | 80 | 85 | 90 | 95 | 100 | 0 |
| 12 | 40 | 20 | 10 | 60 | 80 | 90 | 80 | 0 |
| 13 | 100 | 80 | 90 | 100 | 100 | 100 | 100 | 30 |
| 14 | 100 | 100 | 100 | 80 | 100 | 100 | 100 | 10 |
| 15 | 90 | 60 | 60 | 60 | 80 | 60 | 90 | 0 |
| 16 | 80 | 80 | 60 | 40 | 80 | 80 | 90 | 10 |
| 17 | 0 | 0 | 0 | 40 | 50 | 60 | 20 | 0 |
| 18 | 100 | 100 | 100 | 90 | 100 | 100 | 100 | 0 |
| 19 | 0 | 0 | 0 | 100 | 100 | 100 | 25 | 0 |
| 20 | 100 | 60 | 30 | 85 | 100 | 100 | 0 | 0 |
| 21 | 60 | 60 | 60 | 80 | 100 | 100 | 60 | 0 |
| 22 | 25 | 30 | 30 | 0 | — | 20 | 0 | 0 |
| 23 | 100 | 80 | 80 | 85 | 100 | 100 | 0 | 0 |
| 24 | 100 | 60 | 60 | 80 | 100 | 100 | 100 | 0 |
| 25 | 20 | 0 | 0 | 80 | 90 | 95 | 90 | 0 |
| 26 | 100 | 60 | 85 | 85 | 90 | 95 | 100 | 0 |
| 27 | 0 | 0 | 40 | 100 | 100 | 98 | 100 | 5 |

— = Not tested.

The compounds of the present invention are useful as herbicides, and can be applied in a variety of ways at various concentrations. In practice, the compounds herein defined are formulated into herbicidal compositions, by admixture, in herbicidally effective amounts, with the adjuvants and carriers normally employed for facilitating the dispersion of active ingredients for agricultural applications, recognizing the fact that the formulation and mode of application of a toxicant may affect the activity of the materials in a given application. Thus, these active herbicidal compounds may be formulated as granules of relatively large particle size, as wettable powders, as emulsifiable concentrates, as powdery dusts, as solutions or as any of several other known types of formulations, depending upon the desired mode of application. Preferred formulations for pre-emergence herbicidal applications are wettable powders, emulsifiable concentrates and granules. These formulations may contain as little as about 0.5% to as much as about 95% or more by weight of active ingredient. A herbicidally effective amount depends upon the nature of the seeds or plants to be controlled and the rate of application varies from about 0.05 to approximately 25 pounds per acre (0.56 to 28 Kg/ha) preferably from about 0.1 to about 10 pounds per acre (0.112 to 11.2 Kg/ha).

Wettable powders are in the form of finely divided particles which disperse readily in water or other dispersants. The wettable powder is ultimately applied to the soil either as a dry dust or as a dispersion in water or other liquid. Typical carriers for wettable powders include fuller's earth, kaolin clays, silicas and other readily wet organic or inorganic diluents. Wettable powders normally are prepared to contain about 5% to about 95% of the active ingredient and usually also contain a small amount of wetting, dispersing, or emulsifying agent to facilitate wetting and dispersion.

Emulsifiable concentrates are homogeneous liquid compositions which are dispersible in water or other dispersant, and may consist entirely of the active compound with a liquid or solid emulsifying agent, or may also contain a liquid carrier, such as xylene, heavy aromatic naphta, isophorone and other nonvolatile organic solvents. For herbidical application, these concentrates are dispersed in water or other liquid carrier and normally applied as a spray to the area to be treated. The percentage by weight of the essential active ingredient may vary according to the manner in which the composition is to be applied, but in general comprises about 0.5% to 95% of active ingredient by weight of the herbicidal composition.

Granular formulations wherein the toxicant is carried on relatively coarse particles, are usually applied without dilution to the area in which suppression of vegetation is desired. Typical carriers for granular formulations include sand, fullers's earth, bentonite clays, vermiculite, perlite and other organic or inorganic materials which absorb or which may be coated with the toxicant. Granular formulations normally are prepared to contain about 5% to about 25% of active ingredients which may include surface-active agents such as wetting agents, dispersing agents or emulsifiers; oil such as heavy aromatic naphthas, kerosene or other petroleum fractions, or vegetable oils; and/or stickers such as dextrins, glue or synthetic resins.

Typical wetting, dispersing or emulsifying agents used in agricultural formulations include, for example, the alkyl and alkylaryl sulfonates and sulfates and their sodium salts; polyhydroxy alcohols; and other types of surface-active agents, many of which are available in commerce. The surface-active agent, when used, normally comprises from 0.1% to 15% by weight of the herbicidal composition.

Dusts, which are free-flowing admixtures of the active ingredient with finely divided solids such as talc, clays, flours and other organic and inorganic solids which act as dispersants and carriers for the toxicant, are useful formulations for soil-incorporating application.

Pastes, which are homogeneous suspensions of a finely divided solid toxicant in a liquid carrier such as water or oil, are employed for specific purposes. These formulations normally contain about 5% to about 95% of active ingredient by weight, and may also contain small amounts of a wetting, dispersing or emulsifying agent to facilitate dispersion. For application, the pastes are normally diluted and applied as a spray to the area to be affected.

Other useful formulations for herbicidal applications include simple solutions of the active ingredient in a dispersant in which it is completely soluble at the desired concentration, such as acetone, alkylated naphthalenes, xylene and other organic solvents. Pressurized sprays, typically aerosols, wherein the active ingredient is dispersed in finely-divided form as a result of vaporization of a low boiling dispersant solvent carrier, such as the Freons, may also be used.

The phytotoxic compositions of this invention are applied to the plants in the conventional manner. Thus, the dust and liquid compositions can be applied to the plant by the use of power-dusters, boom and hand sprayers and spray dusters. The compositions can also be applied from airplanes as a dust or a spray because they are effective in very low dosages. In order to modify or control growth of germinating seeds or emerging seedlings, as a typical example, the dust and liquid compositions are applied to the soil according to conventional methods and are distributed in the soil to a depth of at least ½ inch (1.27 cms) below the soil surface. It is not necessary that the phytotoxic compositions be admixed with the soil particles since these compositions can also be applied merely by spraying or sprinkling the surface of the soil. The phytotoxic compositions of this invention can also be applied by addition to irrigation water supplied to the field to be treated. This method of application permits the penetration of the compositions into the soil as the water is absorbed therein. Dust compositions, granular compositions or liquid formulations applied to the surface of the soil can be distributed below the surface of the soil by conventional means such as discing, dragging or mixing operations.

The phytotoxic compositions of this invention can also contain other additaments, for example, fertilizers and other herbicides and pesticides, used as adjuvant or in combination with any of the above-described adjuvants. Other phytotoxic compounds useful in combination with the above-described compounds include, for example, 2,4-dichlorophenoxyacetic acids, 2,4,5-trichlorophenoxyacetic acid, 2-methyl-4-chlorophenoxyacetic acid and the salts, esters and amides thereof, triazine derivatives, such as 2,4 - bis(3 - methoxypropylamino) - 6 - methylthio - s - triazine, 2 - chloro - 4 - ethylamino - 6 - isopropylamino) - s - triazine, and 2 - ethylamino - 4 - isopropyl - amino - 6 - methyl - mercapto - s - triazine; urea derivatives, such as 3 - (3,5 - dichlorophenyl) - 1,1 - dimethylurea and 3 - (p - chlorophenyl) - 1,1 - dimethylurea; and acetamides such as N,N-diallyl-α-chloroacetamide; benzoic acids such as 3-amino-2,5-dichlorobenzoic acid; thiocarbamates such as S-propyl N,N-dipropylthiocarbamate, S - ethyl - N,N - dipropylthiocarbamate, S-ethyl cyclohexylethylthiocarbamate and S-ethyl hexahydro - 1Hazepine - 1 - carbothioate; anilines such as 4 - (methylsulfonyl) - 2,6 - dinitro - N,N - substituted aniline, 4 - trifluoromethyl - 2,6 - dinitro - N,N - di - n - propyl aniline, 4 - trifluoromethyl - 2,6 - dinitro - N - ethyl - N - butyl aniline, 2 - [4 - (2,4 - dichlorophenoxy)phenoxy]propanoic acid, 2 - [1 - (ethoxyimino)butyl] - 5 - [2 - ethylthio)propyl] - 3 - hydroxy - 2 - cyclohexene - one, (±) - butyl - 2[4 - [(5 - trifluoromethyl) - 2 - pyridinyl)oxy]phenoxy]propanate, sodium 5 - [2 - chloro - 4 - (trifluoromethyl)phenoxy] - 2 - nitrobenzoate, 3 - isopropyl - 1H - 2,1,3 - benzothiadiazine - 4(3H) - one - 2,2 - dioxide, and 4 - amino - 6 - tert - butyl - 3 - (methylthio) - as - triazin - 5(4H) - one or (4 - amino - 6 - (1,1 - dimethylethyl) - 3(methylthio) - 1,2,4 - triazin - 5(4H) - one). Fertilizers useful in combination with the active ingredients include, for example, ammonium nitrate, urea and superphosphate. Other useful additaments include materials in which plant organisms take root and grow such as compost, manure, humus and sand.

**Claims for the Contracting States: BE CH DE FR GB IT LI NL**

1. A compound having the structural formula

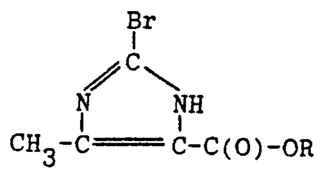

wherein R is methyl or $C_3$—$C_{10}$ alkyl; $C_3$—$C_8$ cycloalkyl; ($C_3$—$C_8$ cycloalkyl)alkyl wherein the alkyl part has 1 to 8 carbon atoms; $C_3$—$C_{10}$ alkenyl; $C_5$—$C_8$ cycloalkenyl; benzyl or mono- or di-substituted benzyl wherein the substituent is $C_1$—$C_4$ alkyl, halogen, nitro or $C_1$—$C_4$ haloalkyl; and salts thereof.

2. A compound of the formula

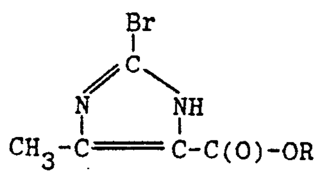

wherein R is allyl or 2-methyl 3-butenyl-1.

3. A compound of the formula

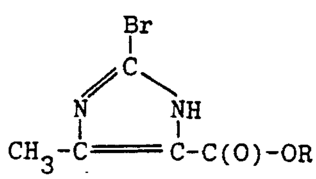

wherein R is methyl or $C_3$—$C_6$ alkyl or $C_4$—$C_6$ cycloalkyl and salts thereof.

4. The compound of Claim 3 wherein R is $C_3$—$C_6$ alkyl.

5. The compound of Claim 4 wherein R is isopropyl, n-butyl, t-butyl, iso-butyl, 1-methylbutyl or methylpropyl.

6. The compound of Claim 3 wherein R is 1-ethylpropyl.

7. The method of controlling undesirable vegetation comprising applying to the area where control is desired an herbicidally effective amount of a compond having the structural formula

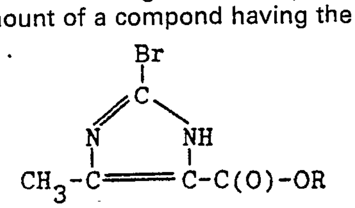

wherein R is $C_1$—$C_{10}$ alkyl; $C_3$—$C_8$ cycloalkyl; ($C_3$—$C_8$ cycloalkyl)alkyl wherein the alkyl part has 1 to 8 carbon atoms; $C_3$—$C_{10}$ alkenyl; $C_5$—$C_8$ cycloalkenyl; benzyl or mono- or di-substituted benzyl wherein the substituent is $C_1$—$C_4$ alkyl, halogen, nitro or $C_1$—$C_4$ haloalkyl; and salts thereof.

8. The method of Claim 7 wherein R is allyl or 2-methyl-3-butenyl-1.

9. The method of Claim 7 wherein R is $C_3$—$C_6$ alkyl or $C_4$—$C_6$ cycloalkyl and salts thereof.

10. The method of Claim 5 wherein R is $C_3$—$C_6$ alkyl.

11. The method of Claim 10 wherein R is isopropyl, n-butyl, t-butyl, iso-butyl, 1-methylbutyl or methylpropyl.

12. The method of Claim 11 wherein R is 1-ethylpropyl.

13. A herbicidal composition comprising a herbicidally active amount of a compound as defined in any of claims 1 to 6.

**Claims for the Contracting State: AT**

1. A method of controlling undesirable vegetation comprising applying to the area where control is desired an herbicidally effective amount of a compound having the structural formula:

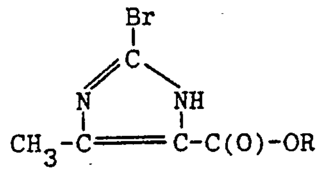

wherein R is $C_1$—$C_{10}$ alkyl; $C_3$—$C_8$ cycloalkyl; ($C_3$—$C_8$ cycloalkyl)alkyl wherein the alkyl part has 1 to 8 carbon atoms; $C_3$—$C_{10}$ alkenyl; $C_5$—$C_8$ cycloalkenyl; benzyl or mono- or di-substituted benzyl wherein the substituent is $C_1$—$C_4$ alkyl, halogen, nitro or $C_1$—$C_4$ haloalkyl; and salts thereof.

2. A method as claimed in Claim 1 characterized in that R is alkyl or 2-methyl-3-butenyl-1.

3. A method as claimed in Claim 1 characterized in that those compounds which are used are those in which R is $C_3$—$C_6$ alkyl.

4. A method as claimed in Claim 1 characterized in that those compounds which are used are those in which R is $C_3$—$C_6$ alkyl or $C_4$—$C_6$ cycloalkyl and salts thereof.

5. A method as claimed in Claim 3 characterized in that R is isopropyl, n-butyl, t-butyl, iso-butyl, 1-methylbutyl or methylpropyl.

6. A method as claimed in Claim 3 characterized in that R is 1-ethylpropyl.

7. A method as claimed in Claim 1 characterized in that the compounds used have the formula:

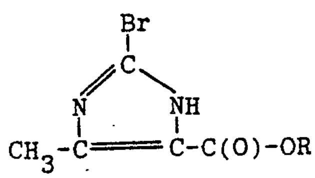

wherein R is methyl or $C_3$—$C_{10}$ alkyl; $C_3$—$C_8$ cycloalkyl; ($C_3$—$C_8$ cycloalkyl)alkyl wherein the alkyl part has 1 to 8 carbon atoms; $C_3$—$C_{10}$ alkenyl; $C_5$—$C_8$ cycloalkenyl; benzyl or mono- or di-substituted benzyl wherein the substituent is $C_1$—$C_4$ alkyl, halogen, nitro or $C_1$—$C_4$ haloalkyl; and salts thereof.

8. A method as claimed in Claim 7 characterized in that R=allyl or 2-methyl-3-butenyl-1.

9. A herbicidal composition comprising a herbicidally active amount of a compound as defined in any of claims 1 to 8 together with an adjuvant.

10. A process for the preparation of compounds as defined in Claim 6 characterized in that one reacts a compound of the formula:

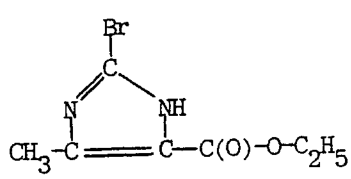

with a compound of the formula:

ROH

in which R has the meaning defined in any of Claims 1 to 8 provided that R is different from an ethyl group

in the presence of a transesterification catalyst, if desired with subsequent conversion to a salt thereof.

11. A process for the preparation of salts of the compounds as defined in any of Claims 1 to 6, characterized in that a Lewis acid or a Lewis base is reacted with the ester.

12. A process as claimed in Claim 10 or Claim 11 in which the compound of formula ROH is one in which R has the meaning given in Claim 7.

**Patentansprüche für die Vertragsstaaten: BE CH DE FR GB IT LI NL**

1. Verbindung mit der Strukturformel

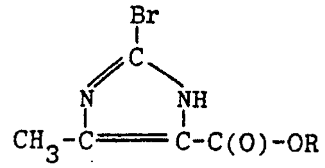

worin R Methyl oder $C_3$—$C_{10}$-Alkyl; $C_3$—$C_8$-Cycloalkyl; ($C_3$—$C_8$-Cycloalkyl)-alkyl, worin der Alkylteil 1 bis 8 Kohlenstoffatome aufweist; $C_3$—$C_{10}$-Alkenyl; $C_5$—$C_8$-Cycloalkenyl; Benzyl oder mono- oder disubstituiertes Benzyl, worin der Substituent $C_1$—$C_4$-Alkyl, Halogen, Nitro oder $C_1$—$C_4$-Halogenalkyl darstellt, bedeutet; und Salze davon.

2. Verbindung der Formel

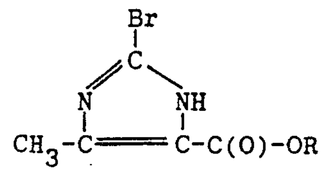

worin R Allyl oder 2-Methyl-3-butenyl-1 bedeutet.

3. Verbindung der Formel

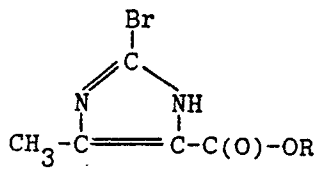

worin R Methyl oder $C_3$—$C_6$-Alkyl oder $C_4$—$C_6$-Cycloalkyl bedeutet, und Salze davon.

4. Verbindung nach Anspruch 3, worin R $C_3$—$C_6$-Alkyl bedeutet.

5. Verbindung nach Anspruch 4, worin R Isopropyl, n-Butyl, t-Butyl, Iso-butyl, 1-Methylbutyl oder Methylpropyl bedeutet.

6. Verbindung nach Anspruch 3, worin R 1-Ethylpropyl bedeutet.

7. Verfahren zur Bekämpfung unerwünschten Pflanzenwachstums, dadurch gekennzeichnet, daß man auf den Bereich, in dem die Bekämpfung erwünscht ist, eine herbicid wirksame Menge einer Verbindung der Strukturformel

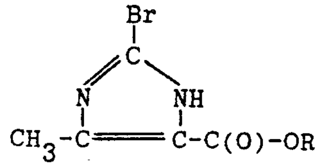

aufbringt, worin R $C_1$—$C_{10}$-Alkyl; $C_3$—$C_8$-Cycloalkyl; ($C_3$—$C_8$-Cycloalkyl)-alkyl, worin der Alkylteil 1 bis 8 Kohlenstoffatome aufweist; $C_3$—$C_{10}$-Alkenyl; $C_5$—$C_8$-Cycloalkenyl; Benzyl oder mono- oder disubstituiertes Benzyl, worin der Substituent $C_1$—$C_4$-Alkyl, Halogen, Nitro oder $C_1$—$C_4$-Halogenalkyl bedeutet, darstellt; und Salze davon.

8. Verfahren nach Anspruch 7, worin R Allyl oder 2-Methyl-3-butenyl-1 bedeutet.

9. Verfahren nach Anspruch 7, worin R $C_3$—$C_6$-Alkyl oder $C_4$—$C_6$-Cycloalkyl bedeutet, und Salze davon.

10. Verfahren nach Anspruch 9, worin R $C_3$—$C_6$-Alkyl bedeutet.

11. Verfahren nach Anspruch 10, worin R Isopropyl, n-Butyl, t-Butyl, Iso-butyl, 1-Methylbutyl oder Methylpropyl bedeutet.

12. Verfahren nach Anspruch 11, worin R 1-Ethylpropyl bedeutet.

13. Herbicides Mittel, enthaltend eine herbicid wirksame Menge einer Verbindung wie in einem der Ansprüche 1 bis 6 definiert.

**Patentansprüche für den Vertragsstaat: AT**

1. Verfahren zur Bekämpfung unerwünschten Pflanzenwachstums, dadurch gekennzeichnet, daß man auf den Bereich, in dem die Bekämpfung erwünscht ist, eine herbicid wirksame Menge einer Verbindung mit der Strukturformel

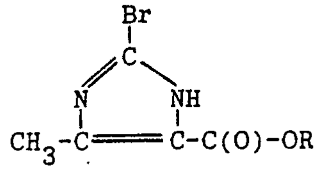

aufbringt, worin R $C_1$—$C_{10}$-Alkyl; $C_3$—$C_8$-Cycloalkyl; ($C_3$—$C_8$-Cycloalkyl)-alkyl, worin der Alkylteil 1 bis 8 Kohlenstoffatome aufweist; $C_3$—$C_{10}$-Alkenyl; $C_5$—$C_8$-Cycloalkenyl; Benzyl oder mono- oder disubstituiertes Benzyl, worin der Substituent $C_1$—$C_4$-Alkyl, Halogen, Nitro oder $C_1$—$C_4$-Halogenalkyl bedeutet, darstellt; und Salze davon.

2. Verfahren wie in Anspruch 1 beansprucht, dadurch gekennzeichnet, daß R Allyl oder 2-Methyl-3-butenyl-1 bedeutet.

3. Verfahren wie in Anspruch 1 beansprucht, dadurch gekennzeichnet, daß diejenigen Verbindungen, welche verwendet werden, diejenigen sind, in denen R $C_3$—$C_6$-Alkyl bedeutet.

4. Verfahren wie in Anspruch 1 beansprucht, dadurch gekennzeichnet, daß diejenigen Verbindungen, welche verwendet werden, diejenigen sind, in denen R $C_3$—$C_6$-Alkyl oder $C_4$—$C_6$-Cycloalkyl bedeutet, und Salze davon.

5. Verfahren wie in Anspruch 3 beansprucht, dadurch gekennzeichnet, daß R Isopropyl, n-Butyl, t-Butyl, Isobutyl, 1-Methylbutyl oder Methylpropyl bedeutet.

6. Verfahren wie in Anspruch 3 beansprucht, dadurch gekennzeichnet, daß R 1-Ethylpropyl bedeutet.

7. Verfahren wie in Anspruch 1 beansprucht, dadurch gekennzeichnet, daß die verwendeten Verbindungen die Formel

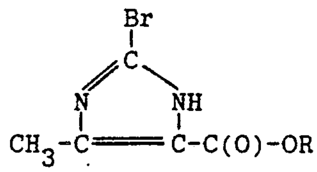

aufweisen, worin R Methyl oder $C_3$—$C_{10}$-Alkyl; $C_3$—$C_8$-Cycloalkyl; ($C_3$—$C_8$-Cycloalkyl)-alkyl, worin der Alkylteil 1 bis 8 Kohlenstoffatome aufweist; $C_3$—$C_{10}$-Alkenyl; $C_5$—$C_8$-Cycloalkenyl; Benzyl oder mono- oder disubstituiertes Benzyl, worin der Substituent $C_1$—$C_4$-Alkyl, Halogen, Nitro oder $C_1$—$C_4$-Halogenalkyl bedeutet, darstellt; und Salze davon.

8. Verfahren wie in Anspruch 7 beansprucht, dadurch gekennzeichnet, daß R Allyl oder 2-Methyl-3-butenyl-1 bedeutet.

9. Herbicides Mittel, enthaltend eine herbicid wirksame Menge einer Verbindung wie in einem der Ansprüche 1 bis 8 definiert zusammen mit einem Adjuvans.

10. Verfahren zur Herstellung von Verbindungen wie in Anspruch 7 definiert, dadurch gekennzeichnet, daß man eine Verbindung der Formel

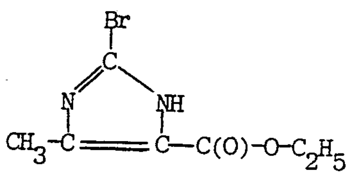

mit einer Verbindung der Formel

ROH

worin R die in einem der Ansprüche 1 bis 8 angegebene Bedeutung mit der Maßgabe aufweist, daß R von einer Ethylgruppe verschieden ist, in Gegenwart eines Umesterungskatalysators umsetzt, gegebenenfalls mit anschließender Umwandlung in ein Salz davon.

11. Verfahren zur Herstellung von Salzen der Verbindungen wie in einem der Ansprüche 1 bis 6 definiert, dadurch gekennzeichnet, daß eine Lewis-Säure oder eine Lewis-Base mit dem Ester umgesetzt wird.

12. Verfahren wie in Anspruch 10 oder Anspruch 11 beansprucht, worin die Verbindung der Formel ROH eine solche ist, in der R die Anspruch 7 angegebene Bedeutung aufweist.

**Revendications pour les Etats contractants: BE CH DE FR GB IT LI NL**

1. Un composé de formule structurelle:

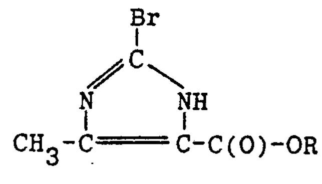

dans laquelle: R représente un radical méthyle ou alkyle en $C_3$ à $C_{10}$, cycloalkyle en $C_3$ à $C_8$, (cycloalkyle en $C_3$ à $C_8$)-alkyle dans lequel la partie alkyle renferme de 1 à 8 atomes de carbone; alkényle en $C_3$ à $C_{10}$; cycloalkényle en $C_5$ à $C_8$; benzyle ou benzyle mono- ou di-substitué dans lequel le substituant représente un radical alkyle en $C_1$ à $C_5$, un atome d'halogène, un groupe nitro ou un radical haloalkyle en $C_1$ à $C_4$; et ses sels.

2. Un composé de formule:

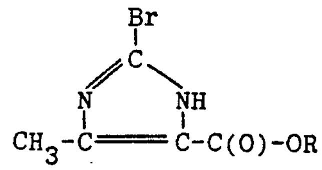

dans laquelle R représente un radical allyle ou 2-méthyl-3-butényl-1.

3. Un composé de formule:

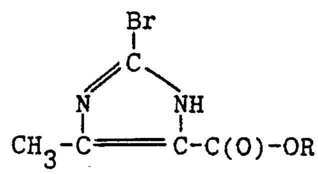

dans laquelle: R représente un radical méthyle ou alkyle en $C_3$ à $C_6$ ou cycloalkyle en $C_4$ à $C_6$, et ses sels.

4. Le composé selon la revendication 3, dans lequel R représente un alkyle en $C_3$ à $C_6$.

5. Le composé selon la revendication 4, dans lequel R représente un radical isopropyl, n-butyle, t-butyle, isobutyle, 1-méthylbutyle ou méthylpropyle.

6. Le composé selon la revendication 3 dans lequel R représente un radical 1-éthylpropyle.

7. La méthode de contrôle d'un végétation indésirable consistant à appliquer, sur la zone où l'on souhaite le contrôle, une quantité efficace en tant qu'herbicide d'un composé de formule structurelle:

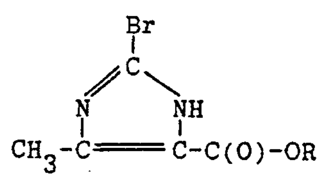

dans laquelle: R représente un radical alkyle en $C_1$ à $C_{10}$; cycloalkyle en $C_3$ à $C_8$; (cycloalkyle en $C_3$ à $C_8$)-alkyle dans lequel la partie alkyle renferme de 1 à 8 atomes de carbone; alkényle en $C_3$ à $C_{10}$; cycloalkényle en $C_5$ à $C_8$; benzyle ou benzyle mono- ou di-substitué dans lequel le substituant représente un radical alkyle en $C_1$ à $C_4$, un atome d'halogène, un groupe nitro ou un radical haloalkyle en $C_1$ à $C_4$; et ses sels.

8. La méthode selon la revendication 7, dans laquelle R représente un radical allyle ou 2-méthyl-3-butény l-1.

9. La méthode selon la revendication 7, dans laquelle R représente un radical alkyle en $C_3$ à $C_6$ ou cycloalkyle en $C_4$ à $C_6$ et leurs sels.

10. La méthode selon la revendication 9, dans laquelle R représente un radical alkyle en $C_3$ à $C_6$.

11. La méthode selon la revendication 10, dans laquelle R représente un radical isopropyle, n-butyle, t-butyle, isobutyle, 1-méthylbutyle ou méthylpropyle.

12. La méthode selon la revendication 11, dans laquelle R représente un radical 1-éthylpropyle.

13. Une composition herbicide comprenant une quantité efficace en tant qu'herbicide d'un composé selon l'une quelconque des revendications 1 à 6.

**Revendications pour l'Etat contractant: AT**

1. Un procédé de contrôle d'un végétation indésirable consistant à appliquer, sur la zone où l'on souhaite le contrôle, une quantité efficace en tant qu'herbicide d'un composé de formule structurelle:

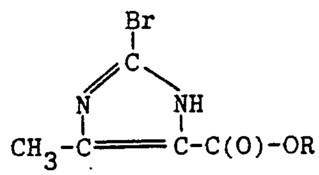

dans laquelle: R représente un radical alkyle en $C_1$ à $C_{10}$; cycloalkyle en $C_3$ à $C_8$; (cycloalkyle en $C_3$ à $C_8$)-alkyle dans lequel la partie alkyle renferme de 1 à 8 atomes de carbone; alkényle en $C_3$ à $C_{10}$; cycloalkényle en $C_5$ à $C_8$; benzyle ou benzyle mono- ou di-substitué dans lequel le substituant représente un radical alkyle en $C_1$ à $C_4$, un atome d'halogène, un groupe nitro ou un radical haloalkyle en $C_1$ à $C_4$; et ses sels.

2. Un procédé selon la revendication 1, caractérisé en ce que R représente un radical allyle ou 2-méthyl-3-butény l-1.

3. Un procédé selon la revendication 1, caractérisé en ce que les composés qui sont utilisés sont ceux dans lesquels R représente un radical alkyle en $C_3$ à $C_6$.

4. Un procédé selon la revendication 1, caractérisé en ce que les composés qui sont utilisés sont ceux dans lesquels R représente un radical alkyle en $C_3$ à $C_6$ ou cycloalkyle en $C_4$ à $C_6$ et leurs sels.

5. Un procédé selon la revendication 3, caractérisé en ce que R représente un radical isopropyle, n-butyle, t-butyle, isobutyle, 1-méthylbutyle ou méthylpropyle.

6. Un procédé selon la revendication 3, caractérisé en ce que R représente un radical 1-éthylpropyle.

7. Un procédé selon la revendication 1, caractérisé en ce que les composés utilisés ont la formule:

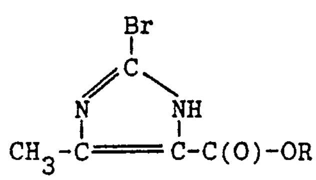

dans laquelle: R représente un radical méthyle ou alkyle en $C_3$ à $C_{10}$, cycloalkyle en $C_3$ à $C_8$, (cycloalkyle en $C_3$ à $C_8$)-alkyle dans lequel la partie alkyle renferme de 1 à 8 atomes de carbone; alkényle en $C_3$ à $C_{10}$; cycloalkényle en $C_5$ à $C_8$; benzyle ou benzyle mono- ou di-substitué dans lequel le substituant représente un radical alkyle en $C_1$ à $C_4$, un atome d'halogène, un groupe nitro ou un radical haloalkyle en $C_1$ à $C_4$; et ses sels.

8. Un procédé selon la revendication 7, caractérisé en ce que R représente un radical allyle ou 2-méthyle-3-butény l-1.

9. Une composition herbicide comprenant une quantité efficace en tant qu'herbicide d'un composé tel que défini dans l'une quelconque des revendications 1 à 8 associé à un adjuvant.

10. Un procédé de préparation de composés selon la revendication 6, caractérisé en ce que l'on fait réagir un composé de formule:

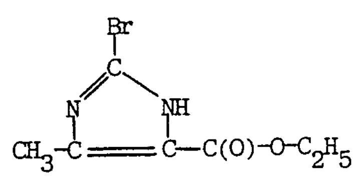

avec un composé de formule:

ROH

dans laquelle R a la signification définie dans l'une quelconque des revendications 1 à 8, pourvu que R soit différent d'un groupe éthyle, en présence d'un catalyseur de transestérification, et si on le souhaite avec conversion ultérieure en un de ses sels.

11. Un procédé de préparation de sels de composés selon l'une quelconque des revendications 1 à 6, caractérisé en ce que l'on fait réagir avec l'ester un acide de Lewis ou une base de Lewis.

12. Un procédé selon la revendication 10 ou 11, dans lequel le composé de formule ROH est un composé dans lequel R a la signification donnée dans la revendication 7.